# EUROPEAN PATENT SPECIFICATION

(11) **EP 3 824 872 B1**
(45) Date of publication and mention of the grant of the patent: **03.07.2024**
(21) Application number: 19838725.0
(22) Date of filing: 05.06.2019
(51) Int. Cl.: A61K 8/44, A61K 8/34, A61Q 19/10

(54) **COSMETIC COMPOSITION FOR INCREASING SKIN EXFOLIATION**
KOSMETISCHE ZUSAMMENSETZUNG ZUR FÖRDERUNG DER HAUTABSCHÄLUNG
COMPOSITION COSMÉTIQUE DESTINÉE À AUGMENTER L'EXFOLIATION DE LA PEAU

(30) Priority: 17.07.2018 KR 20180083069; 30.05.2019 KR 20190063704
(43) Date of publication of application: 26.05.2021
(73) Proprietor: LG Household & Health Care Ltd., Seoul 03184 (KR)
(72) Inventor: AHN, Byung-Jun, Seoul 07795 (KR); KANG, Nae-Gyu, Seoul 07795 (KR); KIM, Jin-Hyun, Seoul 07795 (KR)
(74) Representative: Goddar, Heinz J.
(86) International application number: PCT/KR2019/006806
(87) International publication number: WO 2020/017760

(56) References cited:
- EP-A1- 3 721 864
- WO-A1-2006/013082
- WO-A1-2016/120796
- KR-A- 960 040 351
- KR-A- 20050 113 669
- KR-A- 20080 016 186
- KR-A- 20100 061 881
- KR-A- 20200 043 283
- KR-B1- 100 678 864
- KR-B1- 100 678 865
- KR-B1- 101 165 848
- US-A1- 2002 041 889
- US-A1- 2015 359 732

## Description

### TECHNICAL FIELD

The present disclosure relates to a cosmetic composition for increasing skin exfoliation.

### BACKGROUND ART

The stratum corneum is the outermost layer of skin, and is largely composed of a lipid layer of a lamellar structure including ceramides and keratinocytes. The stratum corneum serves as a protective layer which provides protection from foreign materials and prevents loss of water. Normally, the stratum corneum is composed of 15-20 layers. Skin exfoliation occurs as corneodesmosomes are degraded by the action of serine protease which is activated outside the stratum corneum. This process takes approximately 15-20 days for normal cells. Stratum corneum thickening, or the thickening of the stratum corneum, occurs as exfoliation is delayed due to aging, dryness, acne, etc. of the skin, in addition to genetic disorders such as ichthyosis.

It is known that stratum corneum thickening is generally caused by such factors as decreased skin moisturization, decreased expression or activity of serine protease, ultraviolet rays, reduced cellular activity, etc. In general, such methods as exfoliation using physical scrubbers, etc., exfoliation using chemical agents, enhancement of physiological cellular activity, etc. are used to resolve the stratum corneum thickening. The purpose of exfoliation is to solve the aesthetic problems such as skin roughness or opacity by artificially removing external keratinocytes and replacing them with new keratinocytes from inside, thereby resolving the stratum corneum thickening.

The resolution of stratum corneum thickening through increased exfoliation also provides additional effects such as removal of fine wrinkles, removal of blemish, improvement of skin roughness, etc. A variety of products are used for this purpose. Representative examples include alpha hydroxy acids (AHAs), beta hydroxy acids (BHAs) and poly hydroxy acids (PHAs). AHA, BHA and PHA enter the stratum corneum and release hydrogen ions, thereby weakening the bond of corneodesmosomes and leading to exfoliation. Since they enter the stratum corneum easily and deliver hydrogen ions well at low pH, low pH is preferred. Since side effects such as skin pricking, itchiness, erythema, etc. are caused at low pH, researches are being conducted to overcome these side effects. If the pH is adjusted to weakly acidic or neutral pH to alleviate the skin irritation side effects, the activity is decreased by 80% or more and their efficacies are offset due to chemical modification, making it difficult to provide the effect of exfoliation. Therefore, a new exfoliating agent which is capable of exhibiting exfoliating effect even at weakly acidic or neutral pH, comparable to that of AHA under acidic condition, is necessary.

The inventors of the present disclosure have identified in Korean Patent Application No. 10-2017-0165268 that a composition containing serine and carnitine exhibits better efficacy under neutral or weakly acidic condition than AHA of the same concentration and can be used as a superior novel exfoliation material with relatively little or no irritation.

### DISCLOSURE

### Technical Problem

The present disclosure is directed to providing a cosmetic material which is capable of exhibiting exfoliation-increasing effect reliably with no irritation by selecting the composition of the cosmetic material such that the affinity of an exfoliating agent to keratin-binding proteins can be retained.

The present disclosure is also directed to solving the problem of decreased exfoliating effect occurring when serine or carnitine having better effect than the existing exfoliation material is used for a cosmetic material formulation at weakly acidic to neutral pH.

More specifically, the present disclosure is directed to providing a cosmetic composition for increasing exfoliation which contains serine or carnitine and contains substantially no humectant ingredient having a polyol group or contains the humectant ingredient below a predetermined concentration.

### Technical Solution

The solution is disclosed in the appended claims.

According to an aspect of the present disclosure, the polyol humectant of the cosmetic composition for increasing exfoliation of the present disclosure is one or more selected from the group consisting of glycerin, dipropylene glycol, butylene glycol, glycereth-26, methyl gluceth-20 and pentylene glycol.

According to an aspect of the present disclosure, the exfoliating agent of the cosmetic composition for increasing exfoliation of the present disclosure is contained in an amount of 0.001-20 wt% based on the total weight of the composition.

According to an aspect of the present disclosure, the polyol humectant of the cosmetic composition for increasing exfoliation of the present disclosure is present in an amount of more than 0 wt% but less than or equal to 2 wt% based on the total weight of the composition

According to an aspect of the present disclosure, the pH of the cosmetic composition for increasing exfoliation of the present disclosure is 5-8.

### Advantageous Effects

A cosmetic composition of the present disclosure can exhibit exfoliation-increasing effect reliably as the affinity of an exfoliating agent to keratin-binding proteins is maintained. In particular, the cosmetic composition for exfoliation which contains serine or carnitine, which provides superior exfoliating effect at weakly acidic to neutral pH without irritation as compared to the existing materials, exhibits superior effect since the exfoliating effect of serine or carnitine can be retained.

### DESCRIPTION OF DRAWINGS

FIG. 1 shows a result of evaluating the exfoliation of pig skin for combinations of 5% serine and glycerin.
FIG. 2 shows a result of evaluating the exfoliation of pig skin for combinations of 5% carnitine and glycerin.
FIG. 3 shows a result of evaluating the exfoliation of pig skin for combinations of serine, glycerin, dipropylene glycol and 1,3-butylene glycol.
FIG. 4 shows a result of evaluating the exfoliation of pig skin for combinations of 5% serine and DPG at various concentrations.
FIG. 5 shows a result of evaluating the exfoliation of pig skin for combinations of carnitine, glycerin, dipropylene glycol and 1,3-butylene glycol.
FIG. 6 shows a result of investigating exfoliation-inhibiting effect depending on use of a humectant together.
FIG. 7 shows a result of comparing the exfoliation ability of combinations of serine and glycerin depending on the concentration of glycerin.
FIG. 8 shows a result of comparing the exfoliation ability of combinations of carnitine (5%) and glycerin.
FIG. 9 shows a result of comparing the exfoliation ability of combinations of serine (5%) and glycerin.
FIG. 10 shows a result of comparing the exfoliation ability of combinations of carnitine (0.5%) and glycerin.
FIG. 11 shows a result of comparing the exfoliation ability of combinations of serine (0.5%) and glycerin.
FIG. 12 shows visual change after application for 10 days after DHA staining.
FIG. 13 shows a result of measuring TT100% for combinations of serine and glycerin.

### BEST MODE

The present disclosure provides a cosmetic composition for increasing skin exfoliation, which contains an exfoliating agent and contains a polyol humectant in an amount of more than 0 wt% but less than or equal to 2 wt% based on the total weight of the composition. Hereinafter, the present disclosure is described in detail referring to the attached drawings.

The inventors of the present disclosure have attempted to prepare a marketable cosmetic product for increasing exfoliation by adding an exfoliating agent to a cosmetic composition containing various additives such as a surfactant, a fragrance, an antiseptic, a humectant, a thickener, an antioxidant, etc. They have found out that the exfoliation-increasing ability is significantly lower than that expected for the concentration of the exfoliating agent. Through researches, they have found out that the decrease in exfoliation ability is due to the decreased affinity of the exfoliating agent to keratin-binding proteins because of the humectant ingredient, particularly a humectant having a polyol group, among the various additives of the cosmetic material, and have completed the present disclosure by providing a method for solving the problem.

The present disclosure provides a cosmetic composition capable of retaining the effect of an exfoliating agent, particularly serine or carnitine. More specifically, the present disclosure provides a cosmetic composition containing serine or carnitine, which provides superior exfoliating effect at weakly acidic to neutral pH as compared to the existing material, without skin irritation.

It has been found out that the exfoliating effect of serine or carnitine is decreased when it is applied to a formulation. The present inventors have identified that the exfoliating effect becomes decreased rapidly when a humectant having a polyol group, for example, one or more of glycerin, dipropylene glycol (DPG) and 1,3-butylene glycol (BG), is contained in a specific concentration or higher in the composition. Accordingly, the present disclosure provides a cosmetic composition which contains serine or carnitine and contains substantially no polyol humectant or contains the polyol humectant within a range not significantly decreasing the effect of serine or carnitine.

The term in an amount of more than 0 wt% but less than or equal to 2 wt%" means that the polyol humectant is contained 2 wt% or less, specifically 1 wt% or less, more specifically 0.5 wt% or less, more specifically 0.3 wt% or less, further more specifically 0.1 wt% or less, based on the total weight of the composition

A humectant having a polyol group is used in a cosmetic composition as frequently as purified water. In particular, it is important to provide a cosmetic composition for increasing exfoliation, which contains a polyol humectant, since exfoliation products require moisturization more than other products.

Accordingly, in another exemplary embodiment, the present disclosure provides a cosmetic composition for increasing skin exfoliation, which contains a polyol humectant together with an exfoliating agent. The composition of the present disclosure contains the polyol humectant at a content of more than 0 wt% and 0,05 wt% based on the total weight of the composition. These content ranges have critical significance since the exfoliation effect is decreased significantly if the content of the polyol humectant is outside the above ranges.

The term "exfoliation" used in the present disclosure refers to the elimination or removal of dead skin cells from the stratum corneum. The term includes not only the removal from the stratum corneum by applying physical force but also the removal from the stratum corneum by treating with a cosmetic composition without application of physical force.

According to an aspect of the present disclosure, the exfoliating agent of the cosmetic composition for increasing exfoliation of the present disclosure is selected from one or more of serine and carnitine.

According to an aspect of the present disclosure, the exfoliating agent of the cosmetic composition for increasing exfoliation of the present disclosure may be contained in an amount of 0.001-20 wt%, specifically 0.01-17 wt%, more specifically 0.05-15 wt%, based on the total weight of the composition. If the content is less than 0.001 wt%, it may be difficult to achieve the desired effect. And, if it exceeds 20 wt%, the increase in the effect may be unsatisfactory when considering the increase in content.

According to an aspect of the present disclosure, the polyol humectant of the cosmetic composition for increasing exfoliation of the present disclosure may be one or more selected from the group consisting of glycerin, dipropylene glycol (DPG), butylene glycol (BG), glycereth-26, methyl gluceth-20 and pentylene glycol, specifically one or more selected from the group consisting of glycerin, dipropylene glycol and 1,3-butylene glycol.

According to an aspect of the present disclosure, the polyol humectant of the cosmetic composition for increasing exfoliation of the present disclosure is contained in an amount of 2 wt% or less, more specifically 1 wt% or less, more specifically 0.5 wt% or less, more specifically 0.3 wt% or less, further more specifically 0.1 wt% or less, based on the total weight of the composition. If the content is more than 10 wt%, it may be difficult to achieve the exfoliation effect of the exfoliating agent as desired.

According to an aspect of the present disclosure, the pH of the cosmetic composition for increasing exfoliation of the present disclosure may be 5-8, specifically 5-7.5, more specifically 5.5-7.

The cosmetic composition for increasing exfoliation of the present disclosure may further contain, in addition to the above-described ingredients, additional ingredients commonly used in cosmetic materials in consideration of the formulation of the cosmetic material, purpose of use, etc. In addition, the cosmetic composition may contain various additives necessary and suitable for each formulation and may contain known compounds such as an antiseptic, a thickener, a surfactant, a silicone polymer, an oxidation stabilizer, an organic solvent, an antioxidant, an emollient, a pigment, a fragrance, etc. within ranges not negatively affecting the desired effect.

It was confirmed through pig skin exfoliation test and human skin test using DHA staining that the cosmetic composition according to the present disclosure exhibits superior exfoliation effect even in a formulation containing a polyol humectant.

Specifically, it was confirmed through pig skin exfoliation test that the activity of serine or carnitine at neutral or weakly acidic pH of 5-7 is comparable to or better than that of gluconolactone (PHA) or lactic acid (AHA) at pH 4. Therefore, the effect was investigated for different concentrations of the polyol humectant ingredient.

When the exfoliation effect of a cosmetic composition containing serine or carnitine and glycerin, dipropylene glycol or 1,3-butylene glycol as a polyol humectant ingredient was evaluated by pig skin exfoliation test, the exfoliation effect began to decrease rapidly when the polyol humectant content was 0.1 wt% or larger and was decreased to 0%, comparable to that of water which was a negative control group, when the content exceeded 10 wt%. This means that no significant exfoliation ability can be achieved in human skin test.

In addition, the exfoliation effect of a cosmetic composition containing serine or carnitine and glycerin, dipropylene glycol or 1,3-butylene glycol as a polyol humectant ingredient was evaluated by human skin test using DHA staining. There was no significant difference in turnover time (TT100) from that of a non-application group (20 days) when the polyol humectant content was 10% or 50%. But, for the cosmetic composition for increasing exfoliation of the present disclosure, the turnover time was decreased to 17 days or shorter, specifically 14 days or shorter.

Accordingly, since the activity of the exfoliating agent serine or carnitine is decreased by the polyol humectant glycerin, dipropylene glycol or 1,3-butylene glycol, the cosmetic composition of the present disclosure contains substantially no polyol humectant ingredient or contains the polyol humectant ingredient within a range not significantly decreasing the desired effect. That is to say, the exfoliation composition used in the present disclosure contains serine or carnitine and contains an amount not significantly decreasing the desired effect of one or more polyol humectant of glycerin, dipropylene glycol and 1,3-butylene glycol.

Hereinafter, the present disclosure will be described in more detail through examples.

### 1. Preparation of combinations

For testing the exfoliation effect of the present disclosure, combinations (A)-(H) of serine or carnitine and a humectant ingredient having a polyol group were prepared as follows.

**[Table 1]**

| Combination | Exfoliating agent (wt%) | | Humectant having polyol group (wt%) | | | pH |
|---|---|---|---|---|---|---|
| | Serine | Carnitine | Glycerin | Dipropylene | 1,3-Butylene | |
| | | | | glycol (DPG) | glycol (1,3-BG) | |
| A | 0.01-10 | | | | | 5-7.5 |
| B | 0.01-10 | | 0.1-50 | | | 5-7.5 |
| C | 0.01-10 | | | 0.1-50 | | 5-7.5 |
| D | 0.01-10 | | | | 0.1-50 | 5-7.5 |
| E | | 0.01-10 | | | | 5-7.5 |
| F | | 0.01-10 | 0.1-50 | | | 5-7.5 |
| G | | 0.01-10 | | 0.1-50 | | 5-7.5 |
| H | | 0.01-10 | | | 0.1-50 | 5-7.5 |
| I | 0.01-10 | | 0.1-50 | 0.1-50 | 0.1-50 | 5-7.5 |
| J | | 0.01-10 | 0.1-50 | 0.1-50 | 0.1-50 | 5-7.5 |

### Example 1. Evaluation of pig skin exfoliation

The existing clinical evaluation method has the problems that it takes a long time, it is difficult to conduct experiments under various concentration and pH conditions, and it is difficult to conduct experiments for all the combinations of ingredients. Therefore, a new non-clinical method for evaluating exfoliation is necessary.

The exfoliation evaluation method described in Journal of the European Academy of Dermatology and Venereology, 2014, 28, 415-423 was used. Specifically, pig skin was biopsied and a sample to be tested was added. Then, the number of exfoliated cells was measured with a cell counter and was plotted with water as a negative control group (0%) and 10% gluconolactone at pH 4 as a positive control group (100%).

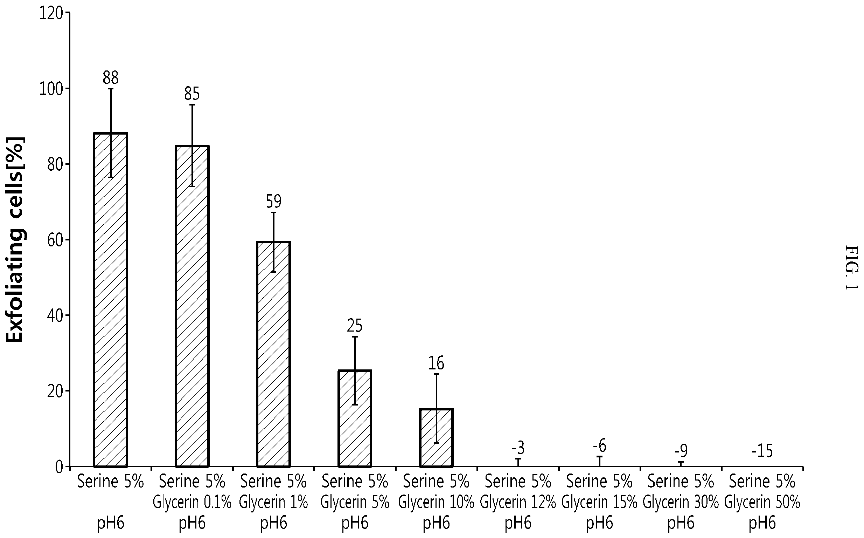

(1) Experimental results for various combinations of 5% serine and glycerin (combinations A and B) are shown in FIG. 1.

**[Table 2]**

| Combination | Exfoliating agent (wt%) | Humectant (wt%) | pH |
|---|---|---|---|
| | Serine | Glycerin | |
| A | 5 | - | 6 |
| B | 5 | 0.1 | 6 |
| B | 5 | 1 | 6 |
| B | 5 | 5 | 6 |
| B | 5 | 10 | 6 |
| B | 5 | 12 | 6 |
| B | 5 | 15 | 6 |
| B | 5 | 30 | 6 |
| B | 5 | 50 | 6 |

The effect of serine was decreased rapidly as glycerin was added. The effect reached 0% when the concentration of glycerin was 10% or higher. Through this, it was confirmed that the effect of serine is decreased as the concentration of glycerin is increased and 10% or less of glycerin should be used to achieve the effect of the exfoliating agent (FIG. 1).

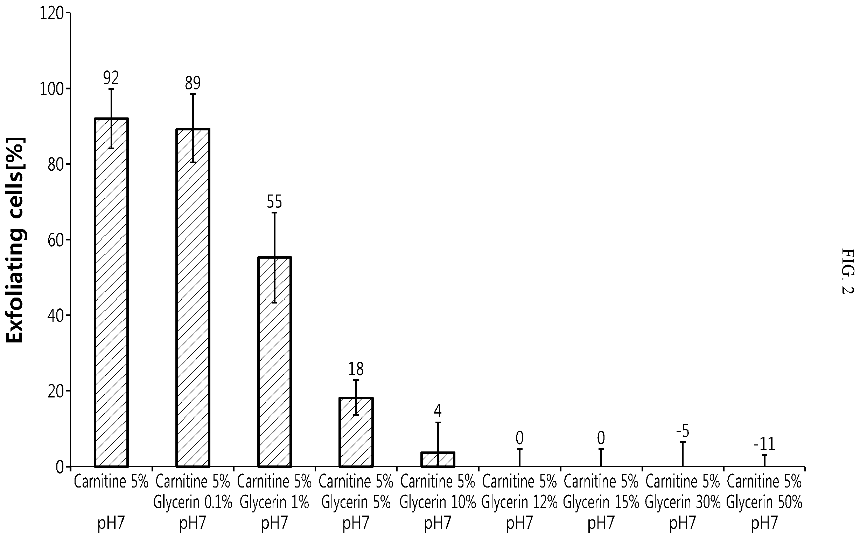

(2) Experimental results for various combinations of 5% carnitine and glycerin (combinations E and F) are shown in FIG. 2.

**[Table 3]**

| Combination | Exfoliating agent (wt%) | Humectant (wt%) | pH |
|---|---|---|---|
| | Carnitine | Glycerin | |
| E | 5 | - | 7 |
| F | 5 | 0.1 | 7 |
| F | 5 | 1 | 7 |
| F | 5 | 5 | 7 |
| F | 5 | 10 | 7 |
| F | 5 | 12 | 7 |
| F | 5 | 15 | 7 |
| F | 5 | 30 | 7 |
| F | 5 | 50 | 7 |

It was confirmed that the exfoliation effect was decreased rapidly when the concentration of glycerin was 5% or higher and reached 0% when the concentration of glycerin was higher than 10 wt% (FIG. 2).

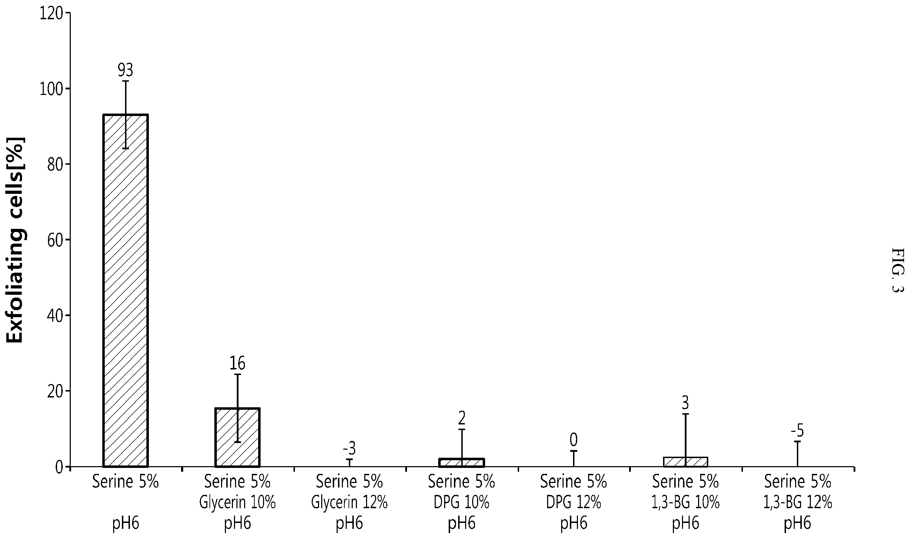

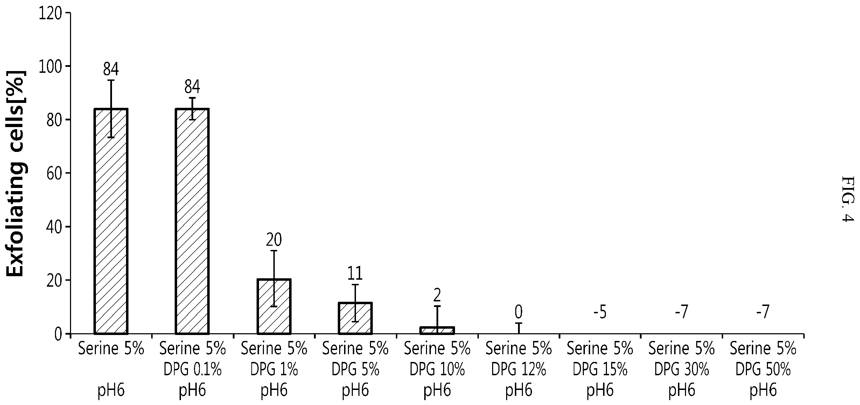

(3) Experimental results for combinations of 5% serine and 10% or 12% glycerin, dipropylene glycol or 1,3-butylene glycol (Table 4; combinations A, B, C and D) are shown in FIG. 3. And, experimental results for 5% serine and DPG at various concentrations (combination D) are shown in FIG. 4.

**[Table 4]**

| Combination | Exfoliating agent (wt%) | Humectant (wt%) | | | pH |
|---|---|---|---|---|---|
| | Serine | Glycerin | Dipropylene | 1,3-Butylene | |
| | | | | | |

| | | | glycol (DPG) | glycol (1,3-BG) | |
|---|---|---|---|---|---|
| A | 5 | | | | 6 |
| B | 5 | 10 | | | 6 |
| B | 5 | 12 | | | 6 |
| C | 5 | | 10 | | 6 |
| C | 5 | | 12 | | 6 |
| D | 5 | | | 10 | 6 |
| D | 5 | | | 12 | 6 |

It was confirmed that the exfoliating effect was decreased when dipropylene glycol or 1,3-butylene glycol was used instead of glycerin. When the concentration of dipropylene glycol or 1,3-butylene glycol exceeded 10%, the exfoliation activity was decreased below that of water (negative control group) and no significant difference could be achieved from the non-application group. Accordingly, it was confirmed again that the exfoliating effect can be achieved when the concentration of one or more humectant having a polyol group, selected from glycerin, dipropylene glycol and 1,3-butylene glycol, in a combination with serine is 10% or lower (FIGS. 3 and 4).

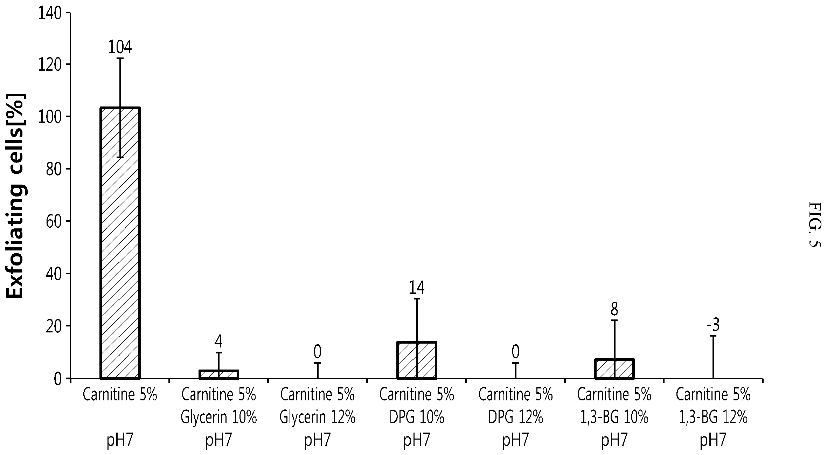

(4) Experimental results for combinations of 5% carnitine and 10% or 12% glycerin, dipropylene glycol or 1,3-butylene glycol (combinations E, F, G and H) are shown in FIG. 5.

**[Table 5]**

| Combination | Exfoliating agent (wt%) | Humectant (wt%) | | pH | |
|---|---|---|---|---|---|
| | Carnitine | Glycerin | Dipropylene glycol (DPG) | 1,3-Butylene glycol (1,3-BG) | |
| E | 5 | | | | 7 |
| F | 5 | 10 | | | 7 |
| F | 5 | 12 | | | 7 |
| G | 5 | | 10 | | 7 |
| G | 5 | | 12 | | 7 |
| H | 5 | | | 10 | 7 |
| H | 5 | | | 12 | 7 |

It was confirmed that the exfoliation activity of carnitine was decreased to the levels where human skin test is meaningless when glycerin, dipropylene glycol or 1,3-butylene glycol was added (FIG. 5).

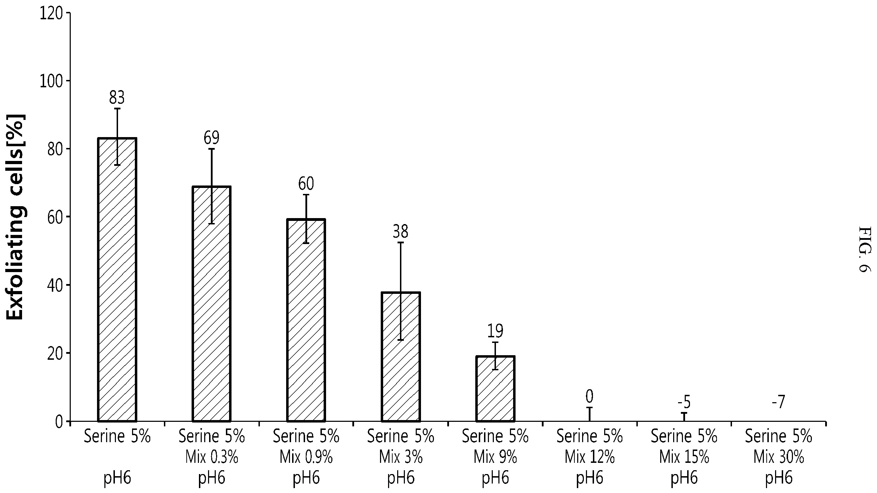

(5) Experimental results for combinations of 5% serine and humectant mixtures (glycerin 1 : DPG 1 : 1,3-BG 1) (combination I) are shown in FIG. 6.

It was confirmed that the exfoliating effect of the exfoliating agent was decreased similarly when it was used together with the mixtures of glycerin, dipropylene glycol and 1,3-butylene glycol. It was also confirmed that the exfoliating effect is achieved when the concentration of the mixture of the humectants having polyol groups was 10% or lower (FIG. 6).

Accordingly, since the combinations with serine or carnitine exhibit exfoliating effect when the content of the one or more humectant having a polyol group, selected from glycerin, dipropylene glycol and 1,3-butylene glycol, is 10 wt% or less, it can be seen that significant exfoliation effect can be achieved when the humectant ingredient is substantially absent or when it is contained below a specific concentration, specifically 10 wt% or lower.

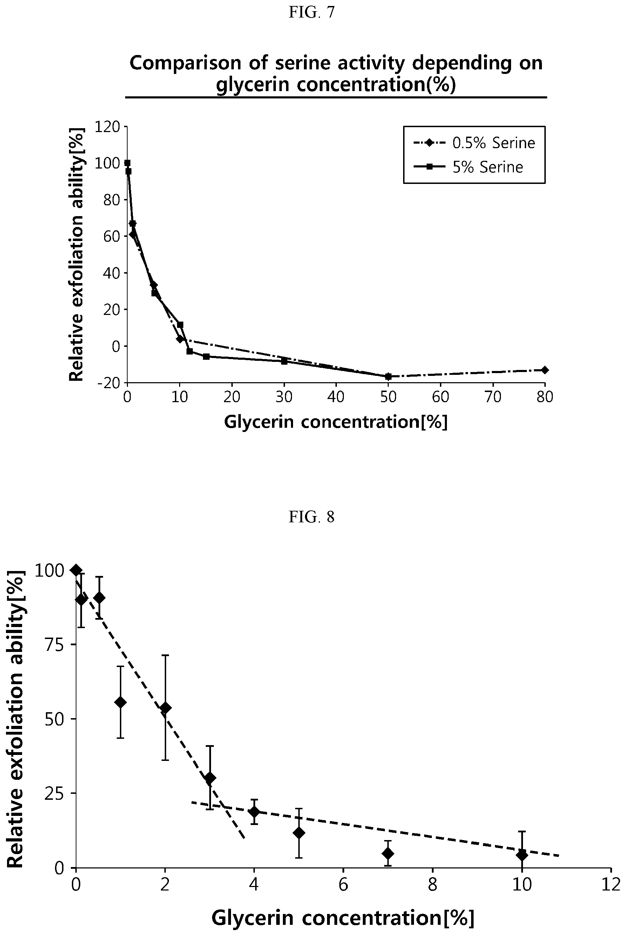

(6) FIG. 7 shows a result of comparing the decrease in exfoliating effect of a 0.5% serine test group and a 5% serine test group depending on the concentration of glycerin, relative to a serine-only positive control group (100%).

It was confirmed that the exfoliating effect was decreased similarly as the glycerin concentration was increased for both the 0.5% serine test group (blue curve in FIG. 7) and the 5% serine test group (orange curve in FIG. 7). Through this, it was confirmed that the concentration of the humectant ingredient having a polyol group in the composition is a major factor in the decrease of exfoliating effect, regardless of the concentration of serine or carnitine. This means that the humectant ingredient having a polyol group should be substantially absent or should be used below a specific concentration, specifically 10% or lower.

In conclusion, it was confirmed that the exfoliation ability of serine and carnitine is affected by the concentration of the humectant having a polyol group, glycerin, dipropylene glycol or 1,3-butylene glycol, and the effect that can be felt by a consumer can be achieved when the humectant ingredient having a polyol group is substantially absent or when its concentration is 10% or lower.

(7) Experiments were conducted to compare relative exfoliation ability for combinations of carnitine (5%) and glycerin at various concentrations. The result is shown in Table 6 and FIG. 8.

**[Table 6]**

| Carnitine wt% | Glycerin wt% | Exfoliation ability % | STD |
|---|---|---|---|
| 5 | 0 | 100 | 7 |
| | 0.1 | 89 | 9 |
| | 0.5 | 91 | 7 |
| | 1 | 55 | 12 |
| | 2 | 53 | 18 |
| | 3 | 30 | 11 |
| | 4 | 19 | 4 |
| | 5 | 12 | 8 |
| | 7 | 5 | 4 |
| | 10 | 4 | 8 |
| | 12 | 0 | 5 |
| | 15 | 0 | 5 |

It was confirmed that, whereas the exfoliation ability was maintained almost constant when the glycerin content was doubled from 1 wt% to 2 wt%, the exfoliation ability was decreased remarkably when the glycerin content was increased to 3 wt%. In addition, whereas the exfoliation ability was even increased when the glycerin content was increased 5-fold from 0.1 wt% to 0.5 wt%, the exfoliation ability was decreased remarkably when the glycerin content was increased to 1 wt%. Through this, the critical significance of the numerical ranges was identified.

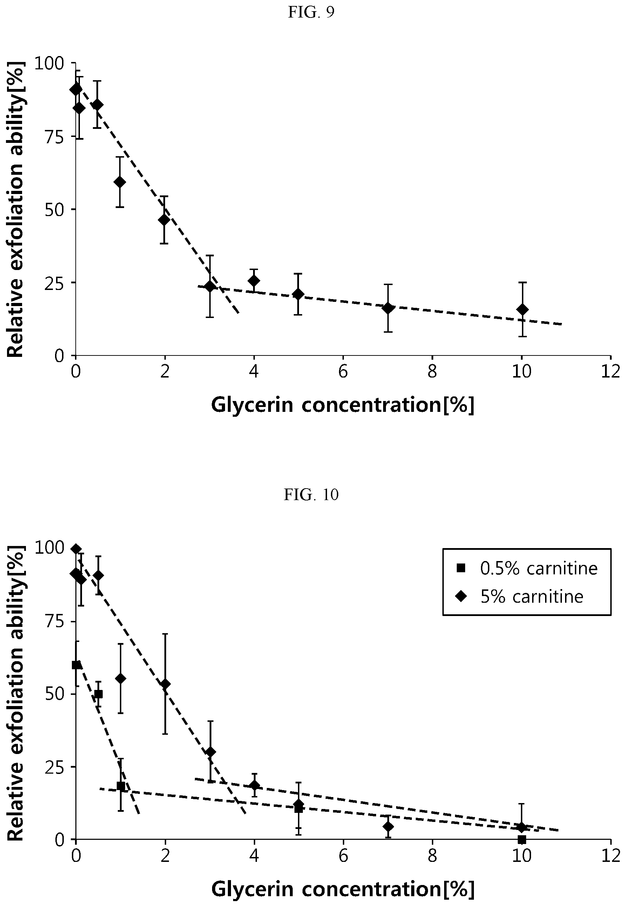

(8) Experiments were conducted to compare relative exfoliation ability for combinations of serine (5%) and glycerin at various concentrations. The result is shown in Table 7 and FIG. 9.

**[Table 7]**

| Serine wt% | Glycerin wt% | Exfoliation ability % | STD |
|---|---|---|---|
| 5 | 0 | 91 | 7 |
| | 0.1 | 85 | 11 |
| | 0.5 | 86 | 8 |
| | 1 | 59 | 9 |
| | 2 | 47 | 8 |
| | 3 | 23 | 11 |
| | 4 | 26 | 4 |
| | 5 | 21 | 7 |
| | 7 | 16 | 8 |
| | 10 | 16 | 9 |
| | 12 | -2 | 4 |
| | 15 | -5 | 7 |

It was confirmed that, whereas the exfoliation ability was maintained almost constant when the glycerin content was doubled from 1 wt% to 2 wt%, the exfoliation ability was decreased remarkably when the glycerin content was increased to 3 wt%. In addition, whereas the exfoliation ability was even increased when the glycerin content was increased 5-fold from 0.1 wt% to 0.5 wt%, the exfoliation ability was decreased remarkably when the glycerin content was increased to 1 wt%. Through this, the critical significance of the numerical ranges was identified.

(9) Experiments were conducted to compare relative exfoliation ability for combinations of carnitine (0.5%) and glycerin at various concentrations. The result is shown in Table 8 and FIG. 10.

**[Table 8]**

| Carnitine wt% | Glycerin wt% | Exfoliation ability % | STD |
|---|---|---|---|
| 0.5 | 0 | 61 | 8 |
| | 0.5 | 50 | 5 |
| | 1 | 18 | 9 |
| | 5 | 11 | 9 |
| | 10 | 0 | 5 |

It was confirmed that superior exfoliation ability comparable to when glycerin was absent was exhibited when the glycerin content was 0.5 wt%; however, the exfoliation ability was decreased remarkably when the glycerin content was increased to 1 wt%. Through this, the critical significance of the numerical range was identified.

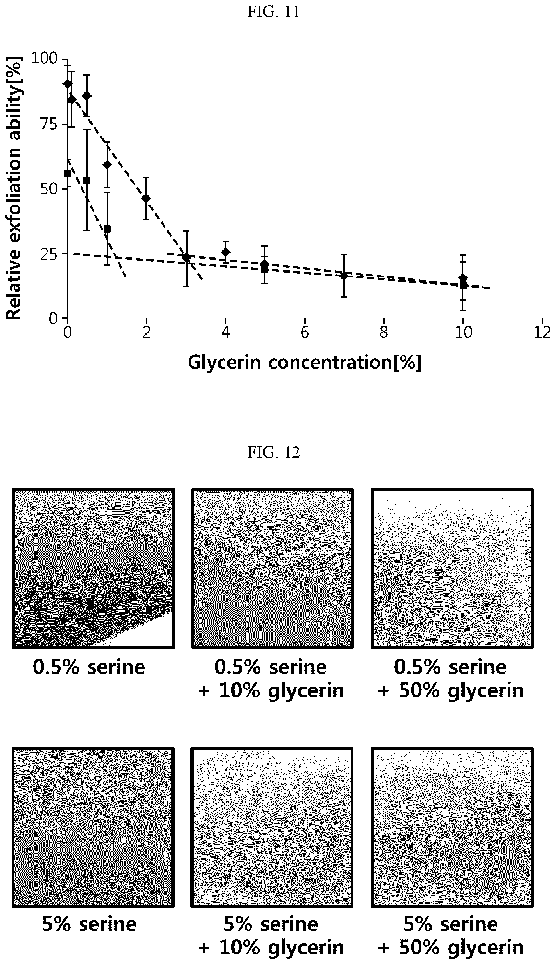

(10) Experiments were conducted to compare relative exfoliation ability for combinations of serine (0.5%) and glycerin at various concentrations. The result is shown in Table 9 and FIG. 11.

**[Table 9]**

| Serine wt% | Glycerin wt% | Exfoliation ability % | STD |
|---|---|---|---|
| 0.5 | 0 | 56 | 5 |
| | 0.5 | 53 | 20 |
| | 1 | 34 | 14 |
| | 5 | 19 | 5 |
| | 10 | 13 | 9 |

It was confirmed that, whereas superior exfoliation ability comparable to when glycerin was absent was exhibited when the glycerin content was 0.5 wt%, the exfoliation ability was decreased remarkably when the glycerin content was increased to 1 wt%. Through this, the critical significance of the numerical range was identified.

### Example 2. Evaluation of exfoliation turnover enhancement by DHA staining

DHA staining for evaluating the speed of exfoliation turnover was conducted for 10 healthy males aged between 20 and 40 years according to the method described in Dermatology 1993, 186, 133-137. Prior to sample application, the color of the inside forearm and upper arm of the test subjects was measured with Chroma Meter. Then, a 1.5 x 1.5 cm gauze soaked with about 0.4 mL of 10% dihydroxyacetone (DHA) was attached to the inside forearm and upper arm for 8 hours. 48 hours later, the color of the part that was pigmented brown by DHA was compared with that before the sample application. Subsequently, while applying the sample twice a day, the time required to return to the original skin color was measured by measuring the skin color every day with Chroma Meter. TT (turnover time) 100% means the time required for the existing stratum corneum to be completely (100%) replaced with a new stratum corneum. In this experiment, the time required for the skin pigmented by DHA corneum to completely return to the original color was compared by regression analysis. The result was compared with that of the negative control group (20 days).

FIG. 12 shows the visual change after application for 10 days. Upon visual examination, there was no significant difference from the non-application group when the glycerin concentration was 10% or 50%. But, use of serine alone was effective at concentrations of 0.5% and 5%.

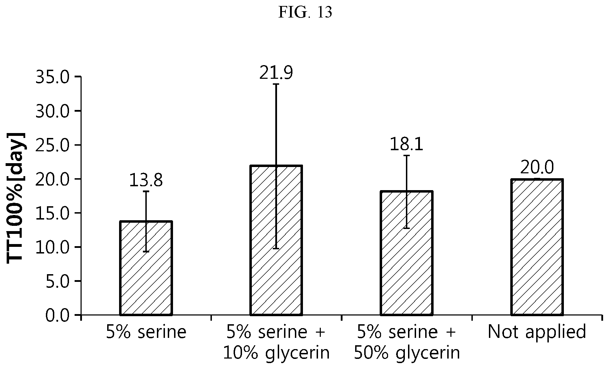

FIG. 13 shows a quantitative result obtained from the measurement with Chroma Meter. When 5% serine was used, TT100% was decreased by 6 days, from 20 days of the non-application group to 14 days. However, there was no significant difference from the non-application group, when the glycerin concentration was 10% or 50%.

No side effect such as erythema, itchiness, burning sensation, etc. was observed during the experimentation period for all the combination groups. Through this, the inhibition of the exfoliation ability in the concentration range determined by the evaluation of exfoliation of pig skin was confirmed also in human skin test. In conclusion, it was confirmed that the exfoliation effect of serine or carnitine that can be felt by a consumer can be achieved when the humectant ingredient having a polyol group, glycerin, dipropylene glycol or 1,3-butylene glycol, is substantially absent or when its concentration is 10% or lower.

## Claims

1. A use of a cosmetic composition comprising one or more exfoliating agent selected from the group consisting of serine and carnitine for increasing skin exfoliation,
wherein the composition comprises a polyol humectant in an amount of more than 0 wt%, but less than or equal to 2 wt%.

2. A method of increasing skin exfoliation, comprising applying a cosmetic composition comprising one or more exfoliating agent selected from the group consisting of serine and carnitine to skin,
wherein the composition comprises a polyol humectant in an amount of more than 0 wt%, but less than or equal to 2 wt%.

3. The use according to claim 1, wherein the composition comprises the polyol humectant in an amount of more than 0 wt%, but less than or equal to 0.5 wt%.

4. The use according to claim 1, wherein the polyol humectant is one or more selected from the group consisting of glycerin, dipropylene glycol, butylene glycol, glycereth-26, methyl gluceth-20 and pentylene glycol.

5. The use according to claim 1, wherein the exfoliating agent is comprised in an amount of 0.001-20 wt% based on the total weight of the composition.

6. The use according to claim 1, wherein the cosmetic composition has a pH of 5-8.

## Patentansprüche

1. Verwendung einer kosmetischen Zusammensetzung, umfassend ein oder mehrere Exfoliationsmittel, ausgewählt aus der Gruppe, bestehend aus Serin und Carnitin, zur Erhöhung der Hautexfoliation,
wobei die Zusammensetzung ein Polyolfeuchthaltemittel in einer Menge von mehr als 0 Gew.-%, aber weniger als oder gleich 2 Gew.-% umfasst.

2. Verfahren zur Erhöhung der Hautexfoliation, umfassend das Auftragen einer kosmetischen Zusammensetzung, umfassend ein oder mehrere Exfoliationsmittel, ausgewählt aus der Gruppe, bestehend aus Serin und Carnitin, auf die Haut,
wobei die Zusammensetzung ein Polyolfeuchthaltemittel in einer Menge von mehr als 0 Gew.-%, aber weniger als oder gleich 2 Gew.-% umfasst.

3. Verwendung nach Anspruch 1, wobei die Zusammensetzung das Polyolfeuchthaltemittel in einer Menge von mehr als 0 Gew.-%, aber weniger als oder gleich 0,5 Gew.-% umfasst.

4. Verwendung nach Anspruch 1, wobei das Polyolfeuchthaltemittel eines oder mehrere ist, ausgewählt aus der Gruppe, bestehend aus Glycerin, Dipropylenglycol, Butylenglycol, Glycereth-26, Methylgluceth-20 und Pentylenglycol.

5. Verwendung nach Anspruch 1, wobei das Exfoliationsmittel in einer Menge von 0,001-20 Gew.-%, bezogen auf das Gesamtgewicht der Zusammensetzung, umfasst ist.

6. Verwendung nach Anspruch 1, wobei die kosmetische Zusammensetzung einen pH-Wert von 5-8 aufweist.

## Revendications

1. Utilisation d'une composition cosmétique comprenant un ou plusieurs agents exfoliants sélectionnés dans le groupe consistant en la sérine et la carnitine pour augmenter l'exfoliation cutanée,
où la composition comprend un humectant de type polyol à une quantité supérieure à 0 % en poids mais inférieure ou égale à 2 % en poids.

2. Méthode permettant d'augmenter l'exfoliation cutanée, comprenant l'application sur la peau d'une composition cosmétique comprenant un ou plusieurs agents exfoliants sélectionnés dans le groupe consistant en la sérine et la carnitine,
où la composition comprend un humectant de type polyol à une quantité supérieure à 0 % en poids mais inférieure ou égale à 2 % en poids.

3. Utilisation selon la revendication 1, où la composition comprend l'humectant de type polyol à une quantité supérieure à 0 % en poids mais inférieure ou égale à 0,5 % en poids.

4. Utilisation selon la revendication 1, où l'humectant de type polyol est un ou plusieurs sélectionnés dans le groupe consistant en les suivants : glycérine, dipropylène glycol, butylène glycol, glycéreth-26, méthylgluceth-20 et pentylène glycol.

5. Utilisation selon la revendication 1, où l'agent exfoliant est compris à une quantité, rapportée au poids total de la composition, dans la plage de 0,001-20 % en poids.

6. Utilisation selon la revendication 1, où la composition cosmétique a un pH de 5-8.
